# EUROPEAN PATENT APPLICATION

(11) **EP 4 258 300 A1**
(43) Date of publication of application: **11.10.2023**
(21) Application number: 21901003.0
(22) Date of filing: 01.12.2021
(51) Int. Cl.: H01F 13/00, H01F 1/06

(54) **METHOD AND DEVICE FOR DEMAGNETIZING AND DISPERSING MAGNETIC PARTICLE**

(30) Priority: 02.12.2020 KR 20200166234
(71) Applicant: Ezdiatech Inc., Chungcheongnam-do 31116 (KR)
(72) Inventor: JUNG, Yong Gyun, Seoul 05698 (KR); HAN, Kiwung, Gyeonggi-do 16902 (KR); MOON, Daekyung, Gunpo-si, Gyeonggi-do 15869 (KR); CHOI, Heelak, Ansan-si, Gyeonggi-do 15345 (KR)
(74) Representative: Prüfer & Partner mbB Patentanwälte · Rechtsanwälte
(86) International application number: PCT/KR2021/018018
(87) International publication number: WO 2022/119315

(57) **Abstract**

The present invention relates to an apparatus for demagnetizing magnetized magnetic particles. The apparatus demagnetizes and disperses magnetic particles to increase the probability of detecting the magnetic particles. The apparatus includes (a) multi-well plate with single or multiple wells arranged, (b) magnetic particles dispensed into the multi-well plate, and (c) a support means supporting the multi-well plate. A demagnetizer capable of moving up and down is provided below the support means. The support means includes a seesaw-type shaker.

## Description

### Technical Field

The present invention relates to a method and apparatus for demagnetizing and dispersing magnetic particles, and more specifically to a method and apparatus for demagnetizing and dispersing magnetized magnetic particles by which the probability of detection of the magnetic particles can be increased.

### Background Art

*In vitro* diagnostics (IVD) is a technique for analyzing samples (e.g., blood, urine, and cells) collected from humans to diagnose human health. Such *in vitro* diagnostic methods include immunodiagnostics, self-blood glucose monitoring, and molecular diagnostics. Particularly, immunodiagnostic techniques can be used to diagnose and track a wide variety of diseases by determining the presence or absence of specific pathogenic proteins based on antigen-antibody reactions. However, the presence of target proteins at concentrations above the limits of detection is a prerequisite for the utilization of immunodiagnostic techniques. Due to this disadvantage, various techniques have been developed to amplify signals generated from low concentrations of proteins.

Particularly, with recent advances in synthetic chemistry and bioscience, various target analytes have appeared in a variety of fields, including new drug development and diagnostics. Several highly sensitive methods have been reported to detect minute amounts of targets using magnetic particles that ensure the generation of signals (Modern magnetic immunoassay: Biophysical and biochemical aspects (2017) Regul. Mech. Biosyst., 9(1), 47-55).

Conventional immunodiagnostic techniques use nanometer-sized magnetic particles surface modified with silica. However, such silica-modified magnetic particles are not effectively extracted or released from wells where immune reactions occur. That is, small-sized magnetic particles are often present in a suspended form in solutions and are thus difficult to separate. For this reason, small-sized magnetic particles are used for qualitative analysis rather than quantitative analysis. Further, conventional analytical methods require a long testing time and involve a complex test procedure because they can test only one kind of magnetic particle at a time and cannot be multiplexed.

It is known that permanent magnets or electromagnets are used to facilitate the movement or reaction of magnetic particles. However, despite the removal of the magnets, the magnetic particles still tend to aggregate due to the residual magnetism. This aggregation makes it impossible to individually observe the particles dispersed during immunological/molecular diagnosis using the magnetic particles, leading to a reduction in detection rate. Further, the aggregation makes it difficult to wash the magnetic particles, and as a result, non-specific signals increase, making it difficult to obtain accurate diagnosis results.

Under these circumstances, new diagnostic methods using magnetic particles are needed in which the magnetism of the magnetic particles is removed to increase the probability of detection of the magnetic particles and by which multiplexed analysis can be performed simultaneously in a short time. However, apparatuses for demagnetizing and dispersing magnetized magnetic particles are required for efficient diagnosis and accurate diagnosis results.

### Detailed Description of the Invention

### Problems to be Solved by the Invention

The present invention has been made to solve the above-described problems and intends to provide a method and apparatus for demagnetizing and dispersing magnetized magnetic particles by which the probability of detection of the magnetic particles can be increased.

### Means for Solving the Problems

One aspect of the present invention provides an apparatus for demagnetizing and dispersing magnetic particles, including: (a) multi-well plate with single or multiple wells arranged; (b) magnetic particles dispensed into the multi-well plate; and (c) a support means supporting the multi-well plate, wherein the support means includes a seesaw-type shaker and a demagnetizer capable of moving up and down is provided below the support means.

In one embodiment, the apparatus may include demagnetization rods disposed at positions corresponding to the wells of the multi-well plate.

In one embodiment, the demagnetizer may descend at a speed of 0.1 to 1000 mm/s after a magnetic force is applied.

In one embodiment, the seesaw-type shaker may turn at an angle in the range of -45 to +45° about its turning axis.

In one embodiment, the magnetic particles may be microrod-like or spherical in shape.

The present invention also provides a diagnostic system including the apparatus.

The present invention also provides a method for demagnetizing and dispersing magnetic particles, including: (i) dispensing magnetic particles into the wells of a multi-well plate; (ii) lifting a demagnetizer such that the uppermost ends of demagnetization rods maintain a predetermined distance from a support means supporting the multi-well plate; (iii) applying power to the demagnetizer to create a magnetic force; (iv) allowing the demagnetizer to descend to demagnetize the magnetic particles in the multi-well plate; and (v) operating a seesaw-type shaker installed in the supporting means to disperse the magnetic particles.

In one embodiment, in step (ii), the distance between the demagnetization rods and the support means may be 0.1 to 100 mm.

In one embodiment, in step (iv), the demagnetizer may descend by 10 to 150 mm.

### Effects of the Invention

The apparatus of the present invention can uniformly disperse magnetic particles in wells while reducing the magnetic force of the magnetic particles, making it easy to observe the fluorescence of the magnetic particles. Therefore, the apparatus of the present invention can increase the efficiency of diagnosis using magnetic particles to obtain more accurate diagnosis results.

In addition, due to its ability to uniformly disperse magnetic particles in wells, the apparatus of the present invention can easily remove foreign matter incorporated in the magnetic particles by washing, extending the service life of the magnetic particles.

### Brief Description of the Drawings

FIG. 1 is a perspective view of an apparatus for demagnetizing and dispersing magnetic particles according to one embodiment of the present invention.
FIG. 2 illustrates (a) side and (b) front views of an apparatus for demagnetizing and dispersing magnetic particles according to one embodiment of the present invention.
FIG. 3 is a partially enlarged view of a seesaw-type shaker of an apparatus for demagnetizing and dispersing magnetic particles according to one embodiment of the present invention.
FIG. 4 illustrates a seesaw-type shaker of an apparatus for demagnetizing and dispersing magnetic particles according to one embodiment of the present invention during operation ((a), (d)) at a neutral position, ((b), (e)) a maximum left angle, and ((c), (f)) a maximum right angle.
FIGS. 5 to 16 show optical observation results obtained in Examples 1-5 and Comparative examples 1-7, respectively, (a) first optical detection results before demagnetization, (b) second optical detection results after demagnetization, and (c) third optical detection results after seesaw-type shaking.

### Mode for Carrying out the Invention

Preferred embodiments of the present invention will now be described in detail. In the description of the present invention, detailed explanations of related art are omitted when it is deemed that they may unnecessarily obscure the essence of the present invention. Throughout the specification, when a certain portion "comprises" or "includes" a certain component, this indicates that the other components are not excluded and may be further included, unless the context specifically requires otherwise.

As the present invention allows for various changes and numerous embodiments, particular embodiments will be illustrated in drawings and described in detail in the written description. However, this is not intended to limit the present invention to modes of practice, and it is to be appreciated that all changes, equivalents, and substitutes that do not depart from the spirit and technical scope of the present invention are encompassed in the present invention.

The present invention is not limited to the illustrated embodiments and may be embodied in various forms. Rather, the disclosed embodiments are provided so that this disclosure will be thorough and complete, and will fully convey the scope of the invention to those skilled in the art. In the drawings, the dimensions, such as widths and thicknesses, of elements may be exaggerated for clarity. The drawings are explained from an observer's point of view. It will be understood that when an element is referred to as being "on" another element, it can be directly on the other element or one or more intervening elements may also be present therebetween. It will be understood by those skilled in the art that various changes in form and details may be made therein without departing from the spirit and scope of the invention as defined by the appended claims. The same reference numerals represent substantially the same elements throughout the drawings.

The terms used herein are merely used to describe embodiments and are not intended to limit the present invention. As used herein, the singular forms "a", "an" and "the" are intended to include the plural forms as well, unless the context clearly indicates otherwise. In the present invention, it is to be understood that the terms such as "including" or "having," etc., are intended to indicate the existence of the features, numbers, steps, operations, actions, components, parts, or combinations thereof disclosed in the specification, and are not intended to preclude the possibility that one or more other features, numbers, steps, operations, actions, components, parts, or combinations thereof may exist or may be added.

On the other hand, terms used herein are to be understood as described below. While such terms as "first" and "second," etc., may be used to describe various elements, such elements must not be limited to the above terms. The above terms are used only to distinguish one element from another. For example, a first element may be referred to as a second element, and likewise a second element may be referred to as a first element.

As used herein, the singular forms "a", "an" and "the" are intended to include the plural forms as well, unless the context clearly indicates otherwise. In the present invention, it is to be understood that the terms such as "including" or "having," etc., are intended to indicate the existence of the features, numbers, steps, operations, actions, components, parts, or combinations thereof disclosed in the specification, and are not intended to preclude the possibility that one or more other features, numbers, steps, operations, actions, components, parts, or combinations thereof may exist or may be added. Respective steps of the methods described herein may be performed in a different order than that which is explicitly described. In other words, the respective steps may be performed in the same order as described, substantially simultaneously, or in a reverse order.

As used herein, the term "and/or" encompasses both combinations of the plurality of related items disclosed and any item from among the plurality of related items disclosed. In the present specification, the description "A or B" means "A", "B", or "A and B."

The present invention is directed to an apparatus for demagnetizing and dispersing magnetic particles, including: (a) multi-well plate 100 with single or multiple wells arranged; (b) magnetic particles dispensed into the multi-well plate; and (c) a support means 200 supporting the multi-well plate 100, wherein the support means 200 includes a seesaw-type shaker 400 and a demagnetizer 300 capable of moving up and down is provided below the support means.

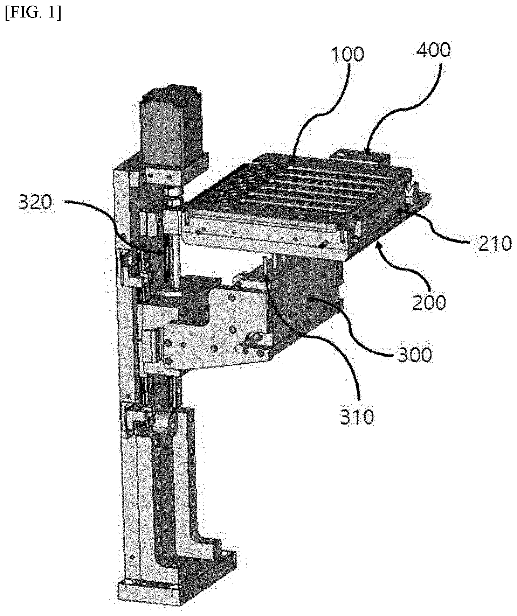

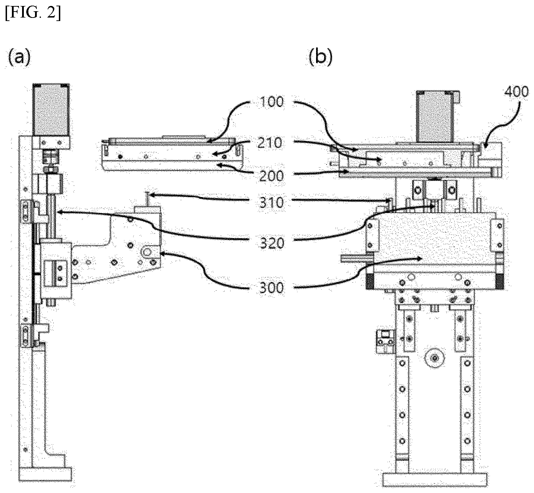

FIG. 1 is a perspective view of the apparatus of the present invention and FIG. 2 illustrates (a) side and (b) front views of the apparatus of the present invention.

Multi-well plates are standard tools for processing and analyzing many samples in chemical, biochemical, and/or biological assays. Multi-well plates may take various forms, sizes, and shapes. Generally, multi-well plates are designed to have standard sizes and shapes and have standard arrangements of wells. The standard arrangements of wells include those found in 96-well plates (12 × 8 array of wells), 384-well plates (24 × 16 array of wells), 1536-well plates (48 × 32 array of wells), and other various commercially available multi-well plates. The multi-well plate used in the present invention is readily compatible with various conventional biological analysis techniques because its dimensions are like those of a commercial multi-well plate. As used herein, the term "multi-well plate" refers to a well plate that has a single well or multiple wells.

Magnetic particles may be dispensed into the multi-well plate 100. Magnetic particles are particles that can be mixed and moved by a magnetic force due to the presence of a material having magnetism. Magnetic particles are commonly used for immunological/molecular diagnosis. The apparatus of the present invention can demagnetize and disperse various magnetic particles having a size of 0.1 nm to 10 mm.

As used herein, the term "demagnetize" means to reduce the magnetism of a magnetic material or to return a magnetic state of a magnetic material from a magnetic state to a non-magnetic state. A magnetic material is magnetized when the electron spins of the atoms of the magnetic material are arranged in a certain direction and is demagnetized by arranging the electron spins of the atoms of the magnetic material in random directions to reduce the magnetism of the magnetic material or to return a magnetic state of the magnetic material to a non-magnetic state.

The apparatus of the present invention may use any magnetic particles whose shapes can be used in diagnostic devices. The apparatus of the present invention can demagnetize magnetic particles having various shapes such as spheres, disks, and microrods, particularly microrod-like magnetic particles.

The magnetic particles may have a core-shell structure. The magnetic particles may consist of a metal core having magnetism therein and a shell which protects the metal core and to which a probe is to be attached. The core is preferably composed of a ferromagnetic material. A ferromagnetic material refers to a material that is strongly magnetized by an external magnetic field and remains magnetized even after the magnetic field is removed. A ferromagnetic material may include iron, nickel, cobalt, or an alloy. Since a ferromagnetic material remains magnetized to some extent even after removal of a magnetic field, it is preferable to quickly remove the magnetic field such that the magnetic particles are dispersed evenly.

The core may be composed of a superparamagnetic material. Even in this case, a small amount of remanence may remain after removal of a magnetic force. Remanence does not have a significant influence on magnetic particles that are used once or twice but accumulate in magnetic particles that are repeatedly used, resulting in aggregation of the magnetic particles. Thus, the apparatus of the present invention removes remanence using the demagnetizer to minimize aggregation of the magnetic particles caused by the remanence.

Particularly, the magnetic particles may be microrod-like in shape. The microrod-like magnetic particles are generally manufactured by cutting core-shell structured microwires to predetermined lengths, with the result that the cores may be exposed on the cut surfaces. Accordingly, when the remanence remains, an attractive force may be generated between the exposed cores, causing the microrods to be lined up as a long chain. This arrangement may reduce the accuracy of the diagnostic device because the types of materials bound to the microrods are classified and analyzed depending on the lengths of the microrods. Thus, the removal of remanence by the demagnetizer 300 can ensure higher detection accuracy, which will be described below.

The support means 200 supports the multi-well plate 100 and can serve to move the multi-well plate 100 while fixing the multi-well plate 100 at a predetermined position. To this end, the support means 200 may include a fixing means 210 fixing the multi-well plate and a moving means (not illustrated) moving the support means 200.

The fixing means 210 serves to fix the multi-well plate 100 to the support means 200. Any means that can fix the multi-well plate 100 at a predetermined position may be used without limitation as the fixing means 210. As a simple example, the fixing means 210 may have a recess through which the multi-well plate is fixedly inserted into the support means 200. Alternatively, the fixing means may use a clip for more reliable fixing of the multi-well plate. Alternatively, the fixing means may have rail portions formed at sides thereof into which wing portions of the multi-well plate 100 are fixedly inserted. The fixing means 210 may temporarily fix the multi-well plate for easy replacement of the multi-well plate and thus it is preferable that the fixing means 210 is easy detachment of the multi-well plate if desired.

The moving means (not illustrated) can move the support means 200 fixed with the multi-well plate 100 up, down, right, and left and can move the multi-well plate 100 to a desired position where a corresponding step is carried out. To this end, the moving means may be disposed to move the support means 200 in three axial directions, more preferably in three orthogonal axial direction (in the X-, Y-, and Z-axis directions). A motor may be installed in the moving means disposed on each axis to move the support means in the corresponding axial direction. More specifically, the support means may be connected to a motor for movement in the X-axis to reciprocate along an X-axis rail. In this case, one or both ends of the X-axis rail are connected to motors for movement in the Y-axis such that the support means reciprocates along a Y-axis rail. The Y-axis rail is connected to a Z-axis motor such that the support means reciprocates along a Z-axis rail. With these connections, the support means can freely move along the X-, Y-, and Z-axes so that the multi-well plate can be accurately placed at a desired position where a corresponding step is carried out. In addition, the support means 200 is installed to be rotatable on the connected shaft, and it is also possible to move in four axial directions.

The support means 200 may include a seesaw-type shaker 400. The magnetic particles may not be uniformly dispersed in the wells even after removal of remanence using the demagnetizer 300, which will be described below. Demagnetization rods of a conventional demagnetizer are inserted into wells to remove the remanence of magnetic particles. When a magnetic force is created in the demagnetization rods, the magnetic particles are bound to the demagnetization rods and their remanence is removed. When the demagnetization rods are removed, the magnetic particles are moved into the wells. Thus, the magnetic particles can be naturally dispersed by the force of gravity while moving from the demagnetization rods into the wells. However, the immersion of the magnetizing rods in the wells reduces the lifetime of the magnetizing rods and a lot of cost is required to prevent unseparated magnetic particles from escaping. To solve these problems, many trials have been made. For example, demagnetization rods are brought into contact with the bottom of wells and the remanence of magnetic particles is then removed. However, the dispersion effect of the magnetic particles due to the attachment and detachment of the magnetic particles disappears, resulting in low detection sensitivity. Particularly, when electromagnets or permanent magnets are used for demagnetization, the magnetic particles are arranged in a row along the direction of the magnetic field. Accordingly, microrod-like particles are impossible to distinguish depending on their length. In contrast, according to the present invention, the demagnetization rods 310 and the demagnetizer 300 are positioned below the support means 200 and the seesaw-type shaker 400 is installed in the support means 200 to evenly disperse magnetic particles free of remanence in the wells.

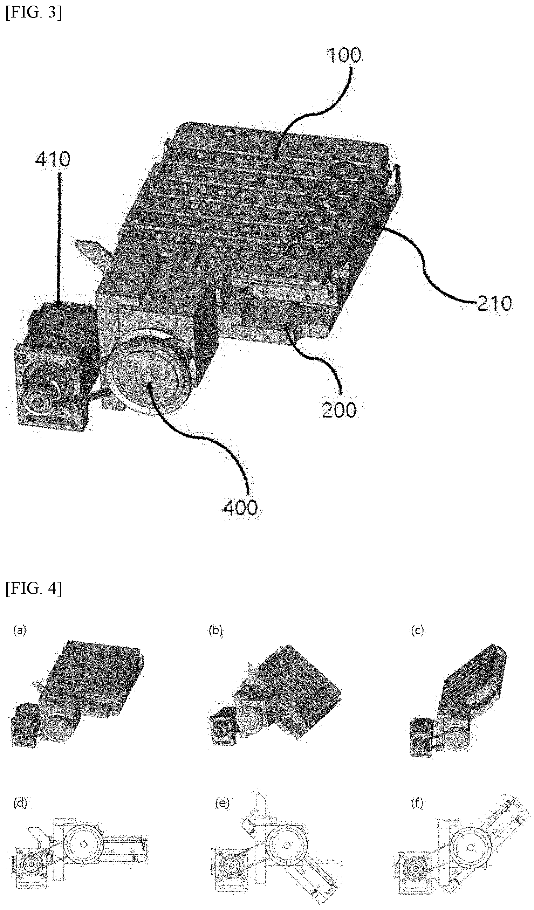

Below is a more detailed discussion of the seesaw-type shaker 400. The seesaw-type shaker 400 may be installed such that the multi-well plate 100 fixed to the support means 200 is turnable at a predetermined angle about the axis of the shaker (see FIG. 3). Since the turning axis of the seesaw-type shaker 400 lies in the same plane as the plane of the multi-well plate 100, the multi-well plate 100 swings at a certain angle about the turning axis like a seesaw when the seesaw-type shaker 400 operates (see FIG. 4). That is, a conventional shaker is installed such that its turning axis is perpendicular to a multi-well plate and stirs the inside of wells. However, the conventional shaker creates a vortex in a certain direction and magnetic particles are arranged along the vortex, limiting their uniform dispersion. In contrast, according to the present invention, the seesaw-type shaker 400 whose turning axis lies in the same plane as the plane of the multi-well plate 100 creates a turbulent flow in the multi-well plate 100, resulting in more uniform and random dispersion of magnetic particles.

The seesaw-type shaker 400 may turn at an angle in the range of -45 to +45° about its turning axis. As the turning angle of the seesaw-type shaker 400 increases, a larger turbulent flow is created in the wells to enable shaking. Particularly, if the turning angle is out of the range defined above, the magnetic particles present in the wells may escape from the wells. It is particularly preferable that when a mixture of the magnetic particles and a liquid is dispensed into the wells, the operating angle of the shaker is adjusted such that the liquid does not flow out of the wells. That is if the turning angle of the seesaw-type shaker 400 exceeds -45 to +45°, the magnetic particles dispensed into the well may flow out and be lost, which is why the cost used for one inspection may rise. For the purpose of preventing magnetic particles from escaping, it is necessary to use a multi-well plate having wells whose size is larger than magnetic particles dispensed into the wells or to reduce the number of magnetic particles dispensed into the wells, which reduces the efficiency of the diagnostic device. Meanwhile, if the turning angle of the seesaw-type shaker is in the range of -10 to +10°, the dispersibility of the magnetic particles by the seesaw-type shaker may be reduced. Accordingly, the seesaw-type shaker is preferably turned at an angle in the range of -45 to +45° about its turning axis. The turning angle is more preferably in the maximum range of -45 to +45° and in the minimum range of -10 to +10°, most preferably in the maximum range of -25 to +25° and in the minimum range of -20 to +20°.

The demagnetizer 300 is provided to remove the remanence of the magnetic particles. In the present invention, it is preferable that AC electromagnets are used to remove the remanence of the magnetic particles.

The magnetic force of the magnetic particles can be usually removed by heating to or above their Curie temperature, applying a large impact or using an attenuated alternating magnetic field.

The Curie temperature of a magnetic material refers to a temperature at or above which the magnetism of the magnetic material changes suddenly. Generally, a magnetic material loses its magnetism when heated to or above its Curie temperature. Also in the present invention, the magnetism of the magnetic particles can be removed by heating the magnetic particles to a temperature equal to or higher than their Curie temperature. However, this cannot be practically achieved in the diagnostic device because the Curie temperatures of most metals exceed 500 °C.

A strong impact on a metal changes the internal atomic arrangement of the metal, leading to a change in the magnetism of the metal. This can be used to remove the remanence of magnetic particles. However, the magnetic particles used in the present invention are vulnerable to impacts due to their core-shell structure and a uniform and strong impact is difficult to apply on the magnetic particles dispensed into the wells, making it difficult to apply an impact in order to remove the remanence of the magnetic particles.

Therefore, in the present invention, it is preferable to remove the remanence of the magnetic particles using an attenuated magnetic field.

The use of an attenuated magnetic field for the removal of the remanence of the magnetic particles will be described below. When a magnetic field is applied to a magnetic material (for example, a ferromagnetic or superparamagnetic material), the atoms of the magnetic material are arranged in a certain direction along the direction of the magnetic field, allowing the magnetic material to have magnetism. Generally, when the magnetic field is removed, the arrangement returns to its original state and the magnetism of the superparamagnetic material should disappear. However, since the arrangement is maintained in the direction of the magnetic field, some of the atoms may have remanence. Particularly, the remanence of the ferromagnetic material tends to last long. Therefore, the remanence of the magnetic material can be demagnetized by eliminating such directionality and making the electron spin arrangement random. The arrangement is usually randomized by transforming the magnetism of electromagnets into a sine wave shape while attenuating the size of the magnetism. Currently, the internal atomic arrangement of the magnetic particles varies depending on a change in the waveform of magnetic force lines of the electromagnets. This waveform is attenuated when transformed into a sine wave shape, and as a result, the internal atomic arrangement may also be rearranged randomly.

However, since such an attenuated magnetic field is complicated to control, an additional controller is essentially required to manage power supply of the electromagnets.

Thus, in the present invention, the remanence of the magnetic particles is removed by a method other than the above-described method. The method using an attenuated magnetic field having a sine wave shape can be applied to not only magnetic particles using a ferromagnetic material but also magnetic particles using a superparamagnetic material as the core material.

A magnetic field can be attenuated by simply adjusting the distance between the demagnetizer 300 and the magnetic particles because the strength of the magnetic field is in inverse proportion to the distance. Accordingly, the demagnetizer 300 used in the present invention can exhibit the same demagnetization performance as conventional demagnetizers.

Therefore, in the present invention, the demagnetizer 300 may be provided to be movable up and down. Preferably, the demagnetizer 300 descends at a speed of 0.1 to 1000 mm/s after the application of a magnetic force. If the speed of the demagnetizer is less than 0.1 mm/s, the attenuation width of the magnetic force lines per hour is small, making it difficult to perform demagnetization. Meanwhile, even if the speed of the demagnetizer exceeds 1000 mm/s, the attenuation width of the magnetic force lines is excessively large, with the result that the magnetic particles may deviate from the magnetic force lines created by the demagnetizer 300 before demagnetization is performed. In conclusion, the demagnetization can be performed smoothly only when the demagnetizer 300 descends at a speed of 0.1 to 1000 mm/s.

The demagnetizer 300 can use DC power instead of AC power, unlike conventional demagnetizers, due to its ability to create a constant magnetic field. Most existing demagnetizers use AC power because they need to generate magnetic force lines in the shape of sine waves, as discussed above. However, since most diagnostic devices use internal DC power, there is a difficulty in that separate power should be supplied for demagnetization. In contrast, since the apparatus of the present invention uses a constant magnetic force to perform demagnetization, it can use the same power source as the internal power source of existing diagnostic devices, which maximizes the efficiency of power management. In order to further increase demagnetization performance, the direction of current supplied to the demagnetizer 300 can also be periodically switched such that a magnetic field having a sine wave shape is supplied, as in conventional demagnetizers. This can also be simplified such that power supplied to the demagnetizer 300 is periodically cut off to create a kind of magnetic field in pseudo-sine wave format.

The demagnetization rods 310 of the demagnetizer 300 may be disposed at positions corresponding to the wells of the multi-well plate 100. If the demagnetizer 300 simply uses rectangular electromagnets, it is difficult to bring the demagnetizer 300 into contact with the bottom of the supporting means 200 below a predetermined distance and it is impossible to supply a uniform magnetic field to each well. For these reasons, the installation of the demagnetization rods 310 at positions corresponding to the wells enables supply of a uniform magnetic field to the wells. Here, it is preferable that the demagnetization rods 310 demagnetize the magnetic particles in the wells with minimal influence on the adjacent wells. It is thus preferable that the demagnetization rods 310 are cylindrical with a diameter equal to or smaller than the wells. If the diameter of the demagnetization rods 310 is larger than that of the wells, the magnetic force lines generated from the demagnetization rods 310 affect the magnetic force lines supplied to the adjacent wells, with the result that destructive interference may occur, making it difficult to smoothly disperse the magnetic particles.

The demagnetization rods 310 may have a length ranging from 0.1 to 100 mm. Within this range, the demagnetizer 300 and the support means 200 can perform demagnetization at a predetermined distance. If the length of the demagnetization rods is less than 0.1 mm, the demagnetizer 300 may approach and come into contact with the support means 200. The distance between the demagnetizer 300 and the support means 200 may be increased to prevent the demagnetizer 300 from approaching and coming into contact with the support means 200. However, the increased distance between the demagnetizer 300 and the support means 200 may cause poor demagnetization efficiency. Meanwhile, if the length of the demagnetization rods exceeds 100 mm, the space for the demagnetizer 300 increases, resulting in an increase in the size of the diagnostic device, which is inefficient.

The demagnetizer 300 may be fixed to a vertically moving means 320 for its vertical movement. The vertically moving means 320 is connected to the demagnetizer 300, like the moving means moving the support means 200. The vertically moving means 320 may include a motor moving the demagnetizer 300 up and down and a rail which is connected to the demagnetizer 300 and along which the demagnetizer 300 is moved up and down. However, the demagnetizer 300 is moved only vertically, unlike the supporting means. Accordingly, it is preferable that the demagnetizer 300 moves one-dimensionally through along one moving means.

The present invention also relates to a diagnostic system including the demagnetization apparatus. The diagnostic system includes magnetic particles and antibodies and probes bound to the magnetic particles. The diagnostic system can detect target analytes bound to the probes through nucleic acid hybridization or antigen-antibody reactions. To this end, secondary antibodies are bound to the target analytes and fluorescent materials or enzymes are bound to the secondary antibodies to detect the target analytes. In the present invention, a plurality of magnetic particles (particularly microrods) having different lengths are used to detect a plurality of target analytes separately or simultaneously. In addition, since the diagnostic system of the present invention performs demagnetization using magnetic particles, it can minimize aggregation of magnetic particles caused by remanence, achieving much higher accuracy than existing diagnostic systems using magnetic particles.

A method for demagnetizing magnetic particles according to the present invention will be described in detail.

The method includes (i) dispensing magnetic particles into the wells of a multi-well plate, (ii) lifting a demagnetizer such that the uppermost ends of demagnetization rods maintain a predetermined distance from a support means supporting the multi-well plate, (iii) applying power to the demagnetizer to create a magnetic force, (iv) allowing the demagnetizer to descend to demagnetize the magnetic particles in the multi-well plate, and (v) operating a seesaw-type shaker installed in the supporting means to disperse the magnetic particles.

In step (i), magnetic particles are prepared and dispensed into the wells of a multi-well plate. The multi-well plate may be prepared only for demagnetization. Alternatively, the multi-well plate may be one that is used for diagnosis. In this case, the multi-well plate is moved to a position where a demagnetizer is provided to perform demagnetization. The magnetic particles may be fresh magnetic particles that are demagnetized before being used for diagnosis. Alternatively, the magnetic particles may be those that are dispensed into the wells, demagnetized, and regenerated.

In step (ii), a demagnetizer is lifted such that the uppermost ends of demagnetization rods maintain a predetermined distance from a support means supporting the multi-well plate. In step (ii), the demagnetization rods are allowed to approach the support means for demagnetization. Here, it is preferable that power supplied to the demagnetizer and the demagnetization rods is cut off such that the demagnetizer and the demagnetization rods move in a state in which no magnetic field is created. Generally, when a magnetic field approaches a ferromagnetic material and its density increases, the remanence of the ferromagnetic material becomes larger. In step (ii), it is thus preferable that power supplied to the demagnetizer is cut off so as not to increase the remanence of the magnetic particles. The distance between the demagnetization rods and the support means may be 0.1 to 100 mm. If the distance is less than 0.1 mm, the demagnetization rods and the supporting means may be brought into contact with each other during movement or demagnetization, causing their damage. Meanwhile, if the distance exceeds 100 mm, the magnetic particles may not be readily demagnetized by the demagnetizer.

In step (iii), power is applied to the demagnetizer to create a magnetic force. Specifically, power, particularly AC power, is applied to the demagnetizer to allow electromagnets provided in the demagnetizer to create a magnetic field. Here, it is preferable to use the same power as the power supplied to the diagnostic device. It is more preferable to supply a power of 24 V, 3 A.

In step (iv), the demagnetizer is allowed to descend to demagnetize the magnetic particles in the multi-well plate. Specifically, the demagnetizer is moved such that the polarity and strength of the magnetic field supplied to the magnetic particles are changed to demagnetize the magnetic particles. For demagnetization, the demagnetizer preferably descends at a speed of 0.1 to 1000 mm/s, more preferably 10 to 50 mm/s, most preferably 60 mm/s. The demagnetizer preferably descends by 10 to 150 mm, more preferably 40 to 80 mm, most preferably 60 mm. If the demagnetizer descends by less than 10 mm, the magnetic field cannot be reduced to a minimum range, with the result that demagnetization may not be completed, leaving remanence behind. Meanwhile, if the demagnetizer descends by more than 150 mm, only the size of the diagnostic device increases, which is inefficient.

In step (v), a seesaw-type shaker installed in the supporting means is operated to disperse the magnetic particles. In step (v), the demagnetized magnetic particles are additionally dispersed. Even when the demagnetization is completed by the demagnetizer, the position of the magnetic particles in the wells may hardly change. Therefore, it is preferable to uniformly disperse the magnetic particles in the wells with shaking using the seesaw-type shaker.

Preferred embodiments of the present invention will be described in detail with reference to the accompanying drawings such that those skilled in the art can readily practice the invention. In describing the present invention, detailed explanations of a related known function or construction are omitted when it is deemed that they may unnecessarily obscure the essence of the invention. Certain features shown in the drawings are enlarged, reduced or simplified for ease of illustration and the drawings and the elements thereof are not necessarily in proper proportion. However, those skilled in the art will readily understand such details.

### Example 1

Glass-coated microwires were produced in accordance with the disclosure of Korean Patent Publication No. 10-2018-0130436. The glass-coated microwires were cut into magnetic microrods having appropriate sizes. Preferably, the glass-coated microwires were cut such that the length was 400 µm, the diameter of the magnetic metal core was 50 µm, and the thickness of the glass coating was 10 µm. Accordingly, the final magnetic microrods had a diameter of 60 µm and a length of 400 µm.

The microrods were demagnetized using the demagnetization apparatus illustrated in FIG. 1. The experimental procedure was as follows.
1) Dispensing of 30 microrods with sizes of 200 µm, 300 µm, 400 µm, and 500 µm into each well filled with distilled water
2) Insertion of a capped magnetic bar into each well to magnetize the microrods
3) Removal of the magnetic bar, upward and downward movement of the cap two or three times for stirring, and removal of the cap
4) First optical detection
5) Ascent of the demagnetizer such that the distance between the demagnetization rod and the bottom surface of the well was 1 mm
6) Supply of power to the demagnetizer (24 V, 3 A)
7) Descent of the demagnetizer by 60 mm for 2 sec and cutting off of the power to the demagnetizer
8) Second optical detection
9) Operation of the seesaw-type shaker for shaking (turning angle ±23°, repeat once, waiting time 1 sec)
10) Third optical detection

### Example 2

The procedure of Example 1 was repeated except that the demagnetizer was allowed to descend by 140 mm for 2 sec.

### Example 3

The procedure of Example 1 was repeated except that the demagnetizer was allowed to descend by 20 mm for 2 sec.

### Example 4

The procedure of Example 1 was repeated except that the turning angle of the seesaw-type shaker was set to ± 40°.

### Example 5

The procedure of Example 1 was repeated except that the turning angle of the seesaw-type shaker was set to ± 15°.

### Comparative Example 1

The procedure of Example 1 was repeated except that no power was supplied to the demagnetizer.

### Comparative Example 2

The procedure of Example 1 was repeated except that the demagnetization rods were not installed.

### Comparative Example 3

The procedure of Example 1 was repeated except that the seesaw-type shaker was not operated.

### Comparative Example 4

The procedure of Example 1 was repeated except that the demagnetizer was allowed to descend by 250 mm for 2 sec.

### Comparative Example 5

The procedure of Example 1 was repeated except that the demagnetizer was allowed to descend by 5 mm for 2 sec.

### Comparative Example 6

The procedure of Example 1 was repeated except that the turning angle of the seesaw-type shaker was set to ± 60°.

### Comparative Example 7

The procedure of Example 1 was repeated except that the turning angle of the seesaw-type shaker was set to ± 5°.

### Test Examples

According to Examples 1-5 and Comparative Examples 1-7, 30 rod-like ferromagnetic particles having lengths of 200 µm, 300 µm, 400 µm, and 500 µm were mixed together, followed by optical measurements. The ferromagnetic particles before demagnetization were used for first optical measurement, the ferromagnetic particles after demagnetization were used for second optical measurement, and the ferromagnetic particles after demagnetization and seesaw-type shaking were used for third optical measurement. AI was used to recognize the lengths of the magnetic particles from the shapes of the optically observed microrods and count the numbers of the magnetic particles. The results are shown in Table 1.

**[Table 1]**

| | 200 µm | | | 300 µm | | | 400 µm | | | 500 µm | | |
|---|---|---|---|---|---|---|---|---|---|---|---|---|
| | First | Second | Third | First | Second | Third | First | Second | Third | First | Second | Third |
| Example 1 | 15 | 25 | 28 | 19 | 29 | 30 | 14 | 23 | 30 | 12 | 20 | 28 |
| Example 2 | 31 | 27 | 29 | 23 | 27 | 29 | 14 | 26 | 28 | 15 | 26 | 29 |
| Example 3 | 16 | 25 | 29 | 22 | 29 | 30 | 17 | 27 | 27 | 14 | 22 | 27 |
| Example 4 | 20 | 31 | 30 | 25 | 29 | 30 | 19 | 24 | 29 | 20 | 28 | 29 |
| Example 5 | 16 | 26 | 27 | 18 | 24 | 28 | 21 | 26 | 29 | 18 | 27 | 28 |
| Comparative Example 1 | 15 | 16 | 14 | 21 | 20 | 22 | 19 | 20 | 16 | 11 | 12 | 12 |
| Comparative Example 2 | 18 | 24 | 26 | 14 | 29 | 29 | 17 | 22 | 26 | 16 | 19 | 24 |
| Comparative Example 3 | 21 | 26 | 25 | 19 | 24 | 26 | 22 | 23 | 24 | 11 | 19 | 20 |
| Comparative Example 4 | 11 | 15 | 18 | 16 | 20 | 21 | 14 | 16 | 20 | 15 | 19 | 18 |
| Comparative Example 5 | 16 | 17 | 16 | 14 | 18 | 16 | 16 | 20 | 22 | 11 | 16 | 15 |
| Comparative Example 6 | 14 | 19 | 24 | 15 | 18 | 21 | 16 | 24 | 20 | 16 | 20 | 22 |
| Comparative Example 7 | 18 | 21 | 23 | 18 | 24 | 25 | 14 | 22 | 23 | 16 | 22 | 21 |

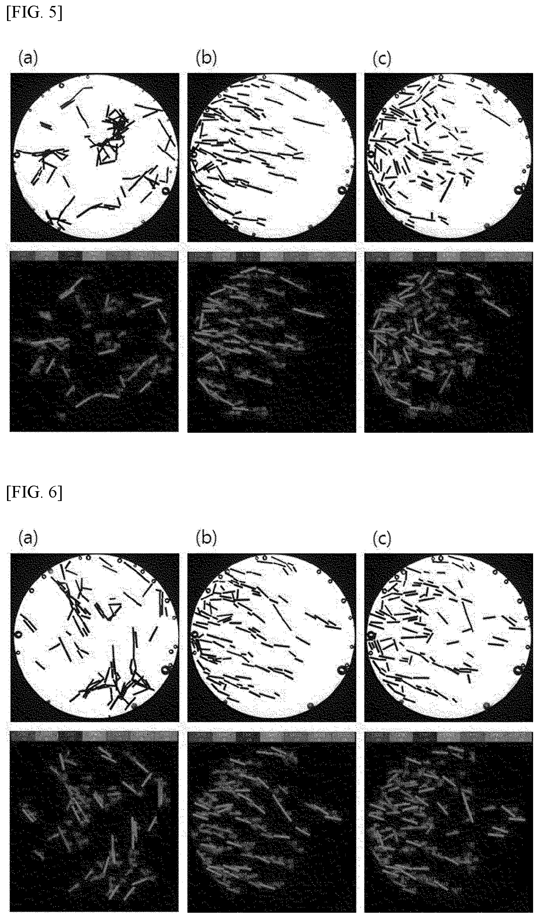

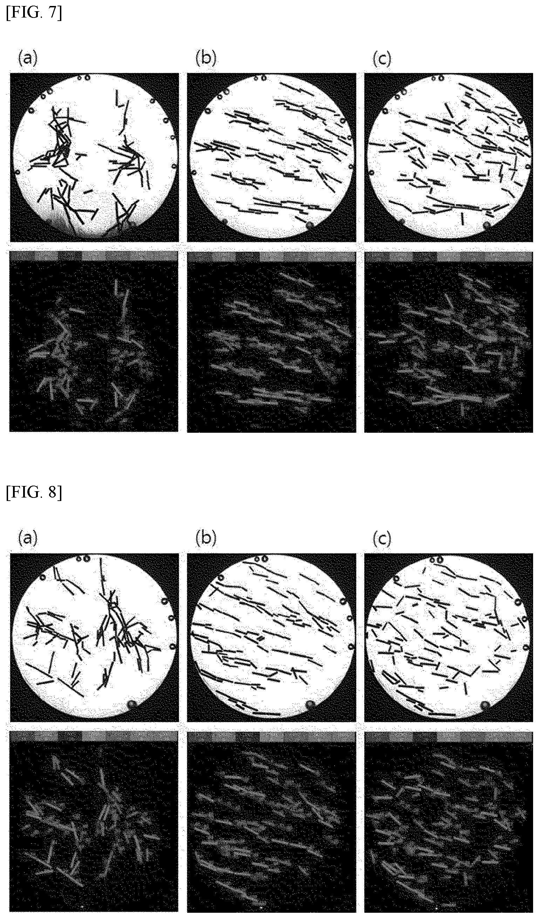

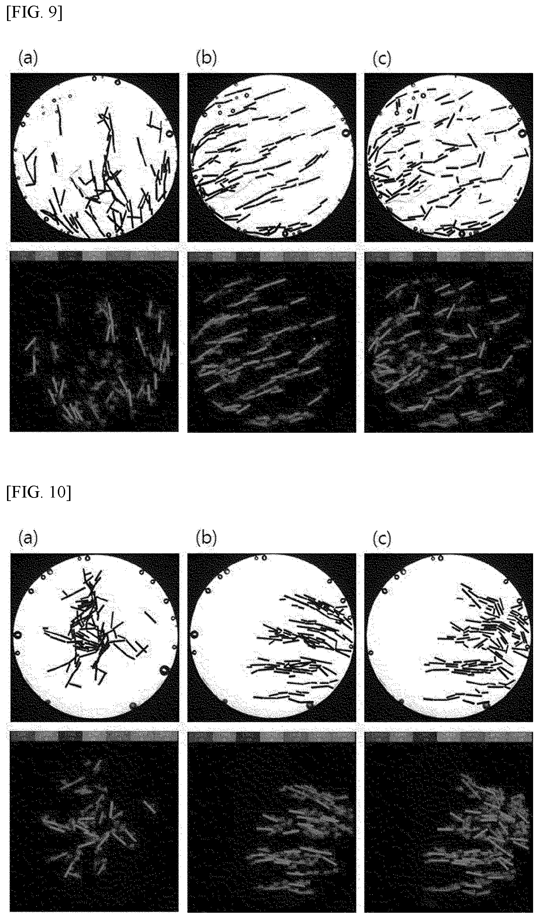

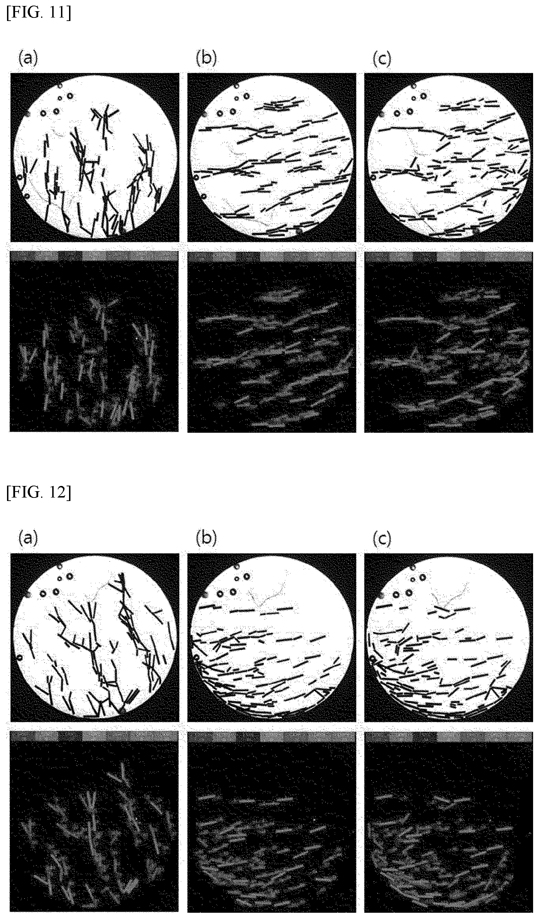

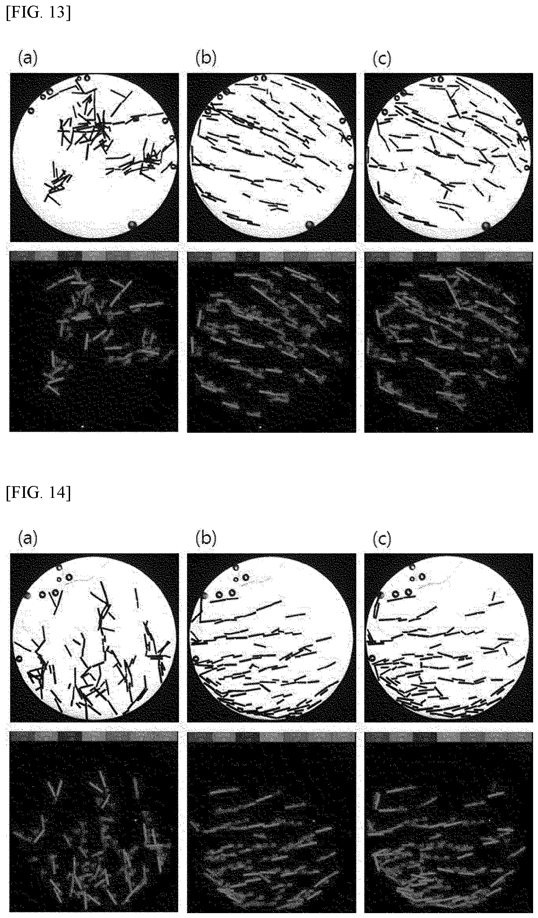

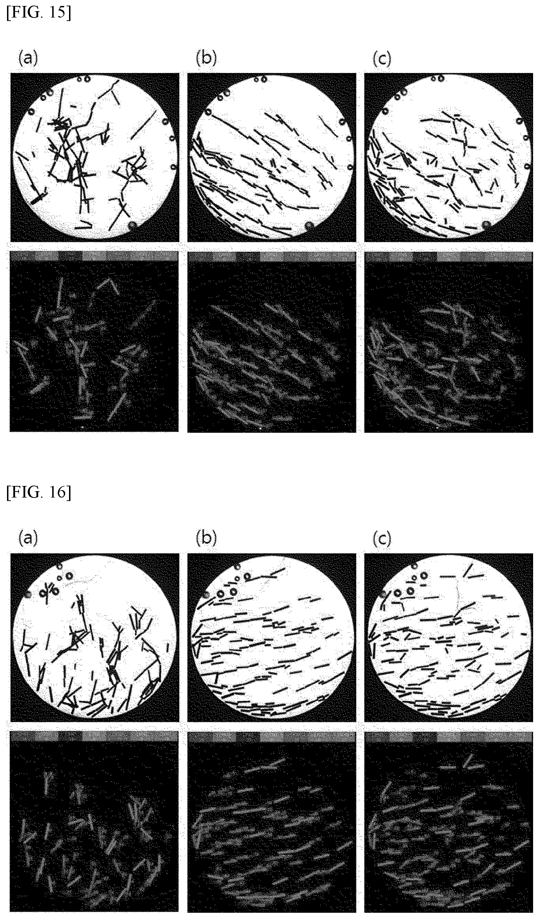

As can be seen from the results in Table 1, the rod-like ferromagnetic particles (microrods) having undergone demagnetization (second optical measurement) and the rod-like ferromagnetic particles having undergone demagnetization and seesaw-type shaking (third optical measurement) could be detected with higher accuracies of ≥ 95% than the rod-like ferromagnetic particles having undergone no demagnetization (first optical measurement). In addition, the rod-like ferromagnetic particles having undergone demagnetization and seesaw-type shaking (third optical measurement) were better demagnetized, achieving higher recognition rate and accuracy, than the rod-like ferromagnetic particles having undergone demagnetization only (second optical measurement) (see FIGS. 5 to 9). In Comparative Example 1 (FIG. 10) where no power was supplied to the demagnetizer, the microrods were not demagnetized and their continuous aggregation was observed in each optical measurement, confirming that the number of the microrods detected was small. Particularly, in the third measurement after seesaw-type shaking, more severe aggregation was observed, leading to a further decrease in the number of microrods detected. These results were also common in Comparative Examples 4 (FIG. 13) and 6 (FIG. 14) where demagnetization was not normally performed. Low demagnetization efficiency was confirmed in Comparative Example 2 (FIG. 11) where the demagnetization rods were not used. No clear improvements could be seen after demagnetization in Comparative Example 3 (FIG. 12) where the seesaw-type shaker was not operated and Comparative Example 7 (FIG. 16) where the seesaw-type shaker was operated with low efficiency.

Finally, the theoretical efficiency in Comparative Example 6 (FIG. 15) where the turning angle of the seesaw-type shaker was increased should be equal to or higher than that in Example 1. However, the distilled water and the magnetic particles dispensed into the wells were lost, resulting in a decrease in the number of observable microrods.

Although the particulars of the present invention have been described in detail, it will be obvious to those skilled in the art that such particulars are merely preferred embodiments and are not intended to limit the scope of the present invention. Therefore, the true scope of the present invention is defined by the appended claims and their equivalents.

## Claims

1. An apparatus for demagnetizing and dispersing magnetic particles, comprising: (a) multi-well plate with single or multiple wells arranged; (b) magnetic particles dispensed into the multi-well plate; and (c) a support means supporting the multi-well plate, wherein the support means comprises a seesaw-type shaker and a demagnetizer capable of moving up and down is provided below the support means.

2. The apparatus according to claim 1, wherein the demagnetizer comprises demagnetization rods disposed at positions corresponding to the wells of the multi-well plate.

3. The apparatus according to claim 1, wherein the demagnetizer descends at a speed of 1 to 1000 mm/s after a magnetic force is applied.

4. The apparatus according to claim 1, wherein the seesaw-type shaker turns at an angle in the range of -45 to +45° about its turning axis.

5. The apparatus according to claim 1, wherein the magnetic particles are microrod-like or spherical in shape.

6. A diagnostic system comprising the apparatus according to any one of claims 1 to 5.

7. A method for demagnetizing and dispersing magnetic particles, comprising: (i) dispensing magnetic particles into the wells of a multi-well plate; (ii) lifting a demagnetizer such that the uppermost ends of demagnetization rods maintain a predetermined distance from a support means supporting the multi-well plate; (iii) applying power to the demagnetizer to create a magnetic force; (iv) allowing the demagnetizer to descend to demagnetize the magnetic particles in the multi-well plate; and (v) operating a seesaw-type shaker installed in the supporting means to disperse the magnetic particles.

8. The method according to claim 7, wherein, in step (ii), the distance between the demagnetization rods and the support means is 0.1 to 100 mm.

9. The method according to claim 7, wherein, in step (iv), the demagnetizer descends by 10 to 150 mm.
